# EUROPEAN PATENT SPECIFICATION

(11) **EP 1 527 090 B1**
(45) Date of publication and mention of the grant of the patent: **11.01.2006**
(21) Application number: 03733634.4
(22) Date of filing: 13.06.2003
(51) Int. Cl.: C07K 14/36, C07K 14/375

(54) **METHOD OF BINDING A COMPOUND TO A SURFACE**
VERFAHREN ZUR BINDUNG EINER VERBINDUNG AN EINE OBERFLÄCHE
PROCEDE POUR COLLER UN COMPOSE SUR UNE SURFACE

(30) Priority: 21.06.2002 EP 02077472
(43) Date of publication of application: 04.05.2005
(73) Proprietor: Applied NanoSystems B.V., 9700 AC Groningen (NL)
(72) Inventor: HEKTOR, Harm, Jan, NL-9725 LD Groningen (NL); RINK, Rick, NL-9717 AG Groningen (NL); SCHOLTMEIJER, Karin, NL-9722 GN Groningen (NL); WEMER, Johannes, NL-9471 AC Zuidlaren (NL); ROGALSKA, Ewa, Maria, F-54000 Nancy (FR); WALCARIUS, Alain, Georges, Ghislain, F-54500 Vandoeuvre-les-Nancy (FR)
(74) Representative: Winckels, Johannes Hubertus F.
(86) International application number: PCT/NL2003/000434
(87) International publication number: WO 2004/000880

(56) References cited:
- WO-A-01/74864
- SCHOLTMEIJER KARIN ET AL: "Surface modifications created by using engineered hydrophobins." APPLIED AND ENVIRONMENTAL MICROBIOLOGY, vol. 68, no. 3, March 2002 (2002-03), pages 1367-1373, XP001120015 March, 2002 ISSN: 0099-2240
- BILEWICZ ET AL: "MODIFICATION OF ELECTRODES WITH SELF-ASSEMBLED HYDROBHOBIN LAYERS" JOURNAL OF PHYSICAL CHEMISTRY. B, MATERIALS, SURFACES, INTERFACES AND BIOPHYSICAL, WASHINGTON, DC, US, vol. 105, 2001, pages 9772-9777, XP002219457 ISSN: 1089-5647 cited in the application
- SCHOLTMEIJER K ET AL: "Fungal hydrophobins in medical and technical applications." APPLIED MICROBIOLOGY AND BIOTECHNOLOGY, vol. 56, no. 1-2, July 2001 (2001-07), pages 1-8, XP002219394 ISSN: 0175-7598
- WOSTEN HAN A B: "Hydrophobins: Multipurpose proteins." ANNUAL REVIEW OF MICROBIOLOGY, vol. 55, 2001, pages 625-646, XP008003420 2001 Annual Reviews 4139 El Camino Way, Palo Alto, CA, 94303-0139, USA ISSN: 0066-4227
- WESSELS J G H: "HYDROPHOBINS: PROTEINS THAT CHANGE THE NATURE OF THE FUNGAL SURFACE" ADVANCES IN MICROBIAL PHYSIOLOGY, ACADEMIC PRESS, LONDON, GB, vol. 38, no. 38, 1997, pages 1-45, XP000999791 ISSN: 0065-2911

## Description

The present invention relates to a method of binding a compound to at least a part of a surface of an object, said method comprising the step of adsorbing a hydrophobin-like substance to said surface.
Classically, hydrophobins are a class of small secreted cysteine-rich proteins of fungi or bacteria that assemble into amphipathic films when confronted with hydrophilic-hydrophobic interfaces. Some hydrophobins form unstable, others extremely stable, amphipathic films. By assembling at a wall-air interface some have been shown to provide for a hydrophobic surface, which has the ultrastructural appearance of rodlets as on aerial hyphae and spores. Some hydrophobins have been shown to assemble into amphipathic films at interfaces between water and oils, or hydrophobic solids, and may be involved in adherence phenomena. It appears that hydrophobins are among the most abundantly produced proteins of fungi, and individual species may contain several genes producing divergent hydrophobins, possibly tailored for specific purposes.
Hydrophobins have now been implicated in various developmental processes, such as formation of aerial hyphae, fruit bodies and conidia, and may play essential roles in fungal ecology, including spore dissemination, pathogenesis and symbiosis.

Hydrophobins fulfill a broad spectrum of functions in fungal growth and development. For instance, they are involved in formation of hydrophobic aerial structures (e.g. aerial hyphae and fruiting bodies) and mediate attachment of hyphae to hydrophobic surfaces resulting in morphogenetic signals. The mechanisms underlying these functions is based on the property of hydrophobins to self-assemble at hydrophilic-hydrophobic interfaces into amphipatic films.
Hydrophobins secreted by submerged hyphae will diffuse in the aqueous environment and may self-assemble at the interface of the medium and the air.
This is accompanied by a huge drop in water surface tension, enabling hyphae to breach the interface and to grow into the air. On the other hand, hydrophobins secreted by hyphae that contact a hydrophobic environment will self-assemble at the hyphal surface. The hydrophilic side of the amphipathic film interacts with the hydrophilic polysaccharides of the cell wall, while the hydrophobic side becomes exposed to the hydrophobic environment. Aerial hyphae and spores thus becom hydrophobic, while hyphae that grow over a hydrophobic subtrate firmly attach to it. Hydrophobins are thus active in the environment of the fungus and at the hyphal surface. Moreover, they also function within the matrix of the cell wall where they somehow influence cell wall composition. In this case monomeric rather than self-assembled hydrophobin seems to be involved.
The best characterised class I hydrophobin is SC3 of *Schizophyllum commune* but, as far as is known by testing, other members of this class have similar properties. Upon contact with hydrophilic-hydrophobic interfaces, SC3 monomers self-assemble into a 10 nm thick amphipathic film. The hydrophilic and hydrophobic sides of the SC3 membrane have water contact angles of 36° and 110°, making these sides moderately hydrophilic (comparable to carbohydrate) and highly hydrophobic (comparable to Teflon), respectively. Interfacial self-assembly of SC3 involves several conformational changes. β-Sheet rich monomers initially adopt a conformation with increases α-helix (α-helix state). SC3 is arrested in this intermediate state at the water-Teflon interface but at the water-air interface the protein proceeds to a form with increased β-Sheet. Initially, this so-called β-Sheet state has no clear ultrastructure (β-Sheet I state) but after a few hours a mosaic of bundles of 10 nm wide rodlets is observed (β-Sheet II state). This ultrastructural change is not accompanied by a detectable change in secondary structure. The transition from the α-helix state to β-Sheet state can also occur at a water-solid interface but has to be induced by increasing the temperature and by adding detergent. Upon self-assembly the properties of hydrophobines change. Hydrophobines in the β-Sheet state is highly surface active, while monomers have no detectable surface activity. Moreover, lectin activity is increased. In addition, the α-helix state form appears to be less stable than the β-Sheet state. Although both forms strongly adhere to hydrophobic surfaces, the α-helix form can be dissociated and converted to the monomeric formation by treatment with cold diluted detergents. In contracts, the conformation of the β-Sheet form and its interaction with the hydrophobic solid is not affected by this treatment. Hydropobins, whether or not chemically or genetically modified, can be used to change the biophysical properties of a surface. In this way, the binding of molecules or cells to surfaces could be controlled. For instance, the binding of pathogenic bacteria to catheter surfaces could be reduced while the binding of human fibroblasts to implant surfaces could be encouraged. Apart from changing the biophysical properties, hydrophobins could be used to attach molecules to surfaces that they do not normally have a high affinity with. Attachment could be achieved by chemical cross-linking after the hydrophobin has been assembled on the surface. For example, proteins could be attached to the mannose residues at the hydrophilic side of assembled SC3 via a Schiff-base reaction. Alternatively, in the case of proteins or peptides, fusion proteins can be made and assembled on the surface of interest. The term "hydrophobin-like substance" as used herein refers to an essentially isolated or purified amphipathic protein capable of coating a surface, rendering a hydrophobic surface essentially hydrophilic, or, vice versa, a hydrophilic surface essentially hydrophobic, and comprises not only hydrophobins as isolated from nature that are essentially free of other fungal components such as carbohydrate polymers such as schyzophylan but include substances that can be obtained by chemically modifying classically known hydrophobins or by genetically modifying hydrophobin genes to obtain genetically modified proteins not at present available from nature, still having the desired amphipathic characteristics. Classically known hydrophobins (see for example WO 96/41882 which also provides guidance to obtain genetically modified hydrophobin-like substances) commonly are proteins with a length of up to 125 amino acids, with a conserved sequence Xₙ-C-X₅₋₉-C-C-X₁₁₋₃₉-C-X₈₋₂₃-C-X₅₋₉-C-C-X₆₋₁₈-C-Xₘ wherein X, of course, represents any amino acid, and n and m, of course, independently represent an integer as disclosed by Wessels et al. (ref. 8).
Most classical hydrophobins contain the eight conserved cysteine residues that form four disulphide bridges. However, when the disulphide bridges of a hydrophobin are reduced by chemical modification and the sulfhydryl groups blocked with for example iodoacetamide the protein assembles in water in the absence of a hydrophilic-hydrophobic interface. The structure is indistinguishable from that of native hydrophobin assembled at the water-air interface.
Apparently, the disulphide bridges of hydrophobins keep monomers soluble in water e.g. within the cell in which they are produced or in the medium, allowing self-assembly at a hydrophilic-hydrophobic interface but are not necessary to provide for its amphipathic character per se.

Class I and class II hydrophobins are known, each at about 100 amino acids in length, having characteristic hydropathy patterns. Most, but not all, contain eight conserved cysteine residues that form intramolecular disulphide bridges. Hydrophobins may be glycosylated, but the characteristic amphipathic properties of these proteins can be solely attributed to their amino acid sequences. Although the amino acid sequences of class II hydrophobins are relatively well conserved, those of the class I hydrophobins show a low homology.
It would be hard, if not impossible, to design universal primers to pick up class I hydrophobin genes by for example polymerase chain reaction.

Indeed, all hydrophobins that have been physically isolated self-assemble at hydrophilic-hydrophobic interfaces into amphipathic membranes. One side of the hydrophobin membrane is moderately to highly hydrophilic (water contact angles below 90°, for example ranging between 22° and 63°), while the other side exposes a surface with water contact angles essentially above 90°, for example ranging between 93° and 140°, for example as hydrophobic as Teflon (polytetrafluorethylene) or paraffin (water contact angle,at about 110°). The membranes formed by class I hydrophobins (e.g. those of SC3 and SC4 of S. *commune*) are highly insoluble [resisting 2% sodium dodecyl sulphate (SDS) at 100°C] but can be dissociated by agents such as formic acid (FA) or trifluoroacetic acid (TFA). In contrast, membranes of the class II hydrophobins cerato-ulmin (CU) of *Ophiostoma ulmi* and cryparin (CRP) of *Cryphonectria parasitica* readily dissociate in 60% ethanol and in 2% SDS, while assembled CU is also known to dissociate by applying pressure or by cooling. Self-assembly of hydrophobins is accompanied by conformational changes. Monomeric class I and class II hydrophobins are rich in β-sheet structure. At the water-air interface, class I hydrophobins attain more β-sheet structure (called the β-sheet state), while at the interface between water and hydrophobic solid, a form with increased α-helix is observed (the α-helical state). The α-helical state seems to be an intermediate of self-assembly, whereas the β-sheet state is likely the stable end-form. At the water-air interface, monomers of class I hydrophobins attain the α-helical state within seconds, but the conversion to the β-sheet state is much slower and takes minutes to hours. At the water-solid interface, the protein also readily attains the α-helical state but is thought to be arrested in this intermediate state. The β-sheet end state can than be reached by applying a combination of heat and diluted detergent. Both forms of the assembled hydrophobin have an amphipathic nature and can be dissociated with TFA, which unfolds the protein.
After removing the solvent and dissolution in water, class I hydrophobins refold to the same monomeric structure that was observed before purification or TFA treatment. However, self-assembly and disassembly of class II hydrophobins can also be repeated even after dissociation of the membrane by TFA. This shows that both classes of hydrophobins are highly resilient to this type of treatment.
The membrane of class I hydrophobins is characterized by a mosaic of bundles of 5-12 nm-wide parallel rodlets. In contrast, rodlets have not been found at surfaces of the assembled class II hydrophobins CFTH1 of *Claviceps fusiformis*, CRP of *C. parasitica*, and HFB1 and HFB2 of *Trichoderma reesei*. Whether the absence of rodlets or the differences in rodlet diameter has any functional significance is not yet known. The rodlets of the class I hydrophobins, SC3 and SC4, of *S. commune* are very similar to the fibrils formed by amyloid proteins. They consist of two tracks of 2-3 protofilaments with a diameter of about 2.5 nm each, have a high degree of β-sheet structure, and interact with the fluorescent dyes Thioflavine T (ThT) and Congo Red. These dyes can be used as probes to discriminate between the alpha-helix state and the beta-sheet state both dyes having a high proensity to satin beta-sheet state but not or only little alpha-helix state or soluble hydrophobin-like substance. In addition, SC3 and amyloid proteins self-assemble via intermediates and only above a critical concentration.
It was suggested that amyloid fibril formation is common to many, if not all, polypeptide chains. However, because formation of amyloid fibrils is accompanied by loss of function or even disease (e.g. Alzheimer's disease), evolution would have selected against the propensity to form such fibrils. Yet, one or two mutation(s) in a protein suffice to considerably increase the tendency to form amyloid fibrils. To our knowledge, hydrophobins are the first example of functional amyloids, with multiple functions in fungal development. Recently, it was found that the four disulfide bridges of the SC3 hydrophobin are essential to prevent the protein from forming the amyloid structures in the absence of a hydrophilic-hydrophobic interface. When the disulphide bridges were reduced and the sulfhydryl groups blocked with iodoacetamide, the protein spontaneously assembled in water. Its structure was then indistinguishable from that of native SC3 assembled at the water-air interface. Apparently, the disulphide bridges of hydrophobins keep monomers soluble in water (e.g. within the cell or in the medium) and thus prevent precocious self-assembly. This would explain why in nature most hydrophobins have eight conserved cysteine residues.
Hydrophobins belong to the most surface-active molecules. With a maximal lowering of the water surface tension from 72 to 24 mJ m⁻² at 50 µg ml⁻¹, SC3 is the most surface-active protein known. Other hydrophobins are also highly surface active. Their surface-lowering activities are at least similar to those of traditional biosurfactants. In contrast to these surfactants, surface activity is not dependent on a lipid conjugate but is solely caused by the amino acid sequence.
Moreover, while the maximal lowering of the surface tension by the traditional surfactants is attained within seconds, it takes minutes to hours in the case of class I hydrophobins. This is explained by the fact that hydrophobins lower the water surface only after self-assembly that is accompanied by conformational changes in the molecule.

Despite the fact that hydrophobins have diverged considerably, their gross properties are similar. This flexibility is also illustrated by the fact that removing 25 out of 31 amino acids preceding the first cysteine residue of the SC3 hydrophobin to generate truncated SC3 by genetic engineering only affected the wettability of the hydrophilic side of the assembled hydrophobin. A most remarkable hydrophobin is the trihydrophobin CFTH1 of *C. fusiformis*. It contains three class II hydrophobin-like units, each preceded by a Gly-Asn-rich repeat and still behaves like other class II hydrophobins. Because of the interfacial self-assembly into amphipathic protein films, hydrophobins can change the wettability of surfaces.

As said, one method to measure wettability is by estimating or measuring the contact angle that a water drop makes with the surface. A large contact angle (>90 deg) indicates a hydrophobic, a small contact angle (<90 deg) a hydrophilic surface. Furthermore, in gas/liquid or liquid/liquid systems, such as in vigorously shaken water or in oil-in-water or water-in-oil dispersions, air bubbles or oil droplets in solution of hydrophobin become coated with an amphipathic film that stabilizes them. Solid/liquid interfaces show the same stabilisation. for example, a sheet of hydrophobic plastic such as teflon (contact angle 110 deg) immersed in hydrophobin becomes coated with a strongly adhering protein film that makes the surface completely wettable (contact angle 48 deg), even after SDS extraction (contact angle 62 deg), and hydrophobin monomers dried down on a hydrophilic surface make the surface hydrophobic.

The classical hydrophobins are i) typically isolated from fungi like Schizophyllum commune (ref. 8) but can now also be made recombinantly; or ii) comprise a polypeptide having at least 40% identity and at least 5% similarity to at least one polypeptide chosen from the group consisting of i) amino acids 29 - 131 of SEQ NO. 1 and ii) amino acids 29 - 133 of SEQ. NO. 2. Such a protein may be derived from a filamentous bacterium, in particular a bacterium capable of forming aerial hyphae such as an Actinomycete, and more specifically the filamentous bacterium may be a Streptomyces species. A Streptomyces species from which the protein may be isolated using standard procedures for the isolation of hydrophobins, is a Streptomyces species which has been transformed with a construct that can be isolated from an E. coli strain which has been deposited on 14 March, 2000 under accession number CBS 102638 with the Centraalbureau voor Schimmelcultures (Oosterstraat 1, P.O. Box 273, 3740 AG Baarn, the Netherlands). This is disclosed in PCT/NL01/00268.
Scholtmeijer et al. (Appl. Environm. Microbiol. 68(3), pp. 1367-1373, 2002) have used fusion proteins of hydrophobins with the cell binding domain of fibronectin (RGD) to provide hydrophobin layers with reactive compounds.

When not using fusion proteins it would appear to be only possible to provide reactive compounds to a hydrophobin layer by covalent coupling. Considering that mannoses are the only glycosylation known for hydrophobins, these are the target of choice for covalently cross-linking compounds to hydrophobin. In particular, Wessels et al. (Advances in Microbial Physiology, 38, pp. 1-45 (1997)) suggest attaching small ligands to a layer of hydrophobin via covalent binding or coupling of amino groups to aldehyde groups on mannose residues (p. 35). The example given therein relates to coupling a protein molecule into a layer of hydrophobin present on a gold surface. Also Schcltmeijer et al. (Appl. Microbiol. Biotechnol. 56, pp. 1-8, 2001) suggest chemical cross-linking of the hydrophobin and the reactive compound, or, as an alternative, fusion proteins. However, for some reactive compounds it is not possible to cross-link or fuse these to hydrophobins without marring the functionality of the reactive compound.
Furthermore, by Bilewicz et al in J. Phys. Chem. B 2001, 105, 9772-9777 of August 2001, a method is described that allows the functionalization of electrode surfaces with small electroactive compounds Q10, azobenzene or Q0, showing that the hydrophobin HYPDPt-1 can act as a molecular glue or as a matrix for electroactive molecules, depending on their structure. However, still a need exists for a method of providing a hydrophobin layer with reactive compounds which are susceptible for changes in their structure for which they are dependent to exert a biological function.

The invention now provides a method of providing a surface of an object with a reactive compound other than a small electroactive compound, said method comprising the steps of coating at least a part of said surface with a hydrophobin-like substance and contacting the compound with the coated hydrophobin-like substance to form a coating comprising said compound in a non-covalently bound form, or at least not covalently bound to a hydrophobin.
A small electroactive compound as defined herein is a compound which undergoes changes in oxidation state, i.e. a redox reaction, and which further has a molecular weight lower than the molecular weight of hydrophobin, particularly a molecular weight of less than 2000 dalton, more particularly less than 1000 dalton. A reactive compound herein is defined as comprising proteins (including peptides and glycoproteins) and nucleic acids.

Here, a method according to the present invention is provided wherein a first (non-covalently attached) reactive compound comprises for example a proteinaceous substance, such as a peptide, but advantageously a polypeptide with a higher molecular weight than a classically known hydrophobin (i.e. >15 kD) which, not-withstanding, becomes non-covalently attached to said coating without essentially losing its reactivity. Surprisingly, substantial non-covalent attachment of such a compound to a surface, whereby preferably reactivity of said compound such as enzymatic activity or its propensity to bind to a ligand or antigen, is maintained, can be achieved by immersing said surface in a solution comprising said compound. Where covalent linking is classically thought to be essentially related to the presence of glycosylated hydrophobin, it is now provided that said surfaces can also be provided with larger molecules when said coating is essentially devoid of mannose residues that would allow for such covalent linking. This facilitates the use of other hydrophobin-like substances for coating object surfaces to which larger molecules may be attached. Where classically one used SC3, which in its natural states is provided with an N-terminal side comprising glycosylated residues, it is also possible to use non-glycosylated substances, such as trSC3 (truncated-SC3) from which said glycosylated N-terminal is absent, but also other hydrophobin-like substances, characterised by an amphipathic protein character, for example those not having all the classically conserved cysteine-residues in place and essentially capable of providing a hydrophobic surface with a hydrophilic face, or vice versa.

In one embodiment, the invention provides an object having at least a part of its surface provided with an amphipathic hydrophobin-like coating (or membrane) wherein said coating is additionally provided with a reactive compound other than a small electroactive compound. Surprisingly, it has been found that said reactive compound may have much larger molecular weights than for example Q10, azobenzene or Q0, non-covalent binding is now provided for a reactive compound that has a molecular weight (MW) larger than 1 kilodalton (kD), preferably larger than 2 kD, more preferably larger than 15 kD, more preferably larger than 50 kD. Furthermore, binding is also provided when said coating is essentially devoid of mannose residues, allowing binding of peptides or polypeptides or other proteinaceous substances independently from covalent cross-linking to mannose residues, leaving many more hydrophobin-like substances available for the provision with a reactive compound than the classically known glycosylated hydrophobins. As said, such binding now allows binding of enzymes, receptors, antibodies, binding molecules per se and other active proteins (or for that matter nucleic acid allowing hybridization) that are not hindered in their (secondary or tertiary) conformational requirements, as is often encountered when using conventional cross-linking, thereby leaving their reactivity intact. The advantage of immobilizing proteins, such as enzymes or antibodies, lies also in often prolonged stability over time but also increased stability at higher temperatures or more extreme pH values are observed.

For another example, an object wherein said reactive compound comprises a nucleic acid preferably comprises a gene chip or DNA (or for that matter RNA or PNA) array, allowing for example gene expression profiling. Nucleic acid that is non-covalently bound to said hydrophobin-like coating allows for improved hybridization protocols.

In general, the invention provides an object having a surface that comprises a hydrophobic/hydrophilic interface and that comprises a reactive compound other than a small electroactive compound, such surface may comprises a liquid/liquid interface, such as an oil/water interface, it may comprises a solid/liquid interface, such as a solid/water interface, or even a solid/solid interface, especially wherein a first solid comprises a hydrophobic surface, and a second solid comprises a hydrophilic surface. The invention provides a method for obtaining an object having at least part of its surface at said interface provided with an amphipathic hydrophobin-like coating wherein said coating is additionally provided with a reactive compound, allowing said compound to remain reactive, that is stable and active, even allowing prolonged storage in an essentially water-deprived or even fully dry form on the coated surface, said method comprising contacting said object with a solution of a hydrophobin-like compound to obtain a coated object and contacting said coated object with a solution containing at least one reactive compound, under conditions favorable for coating said surface and for attaching said reactive compound to said coating. One advantage of using hydrophobin-like substances is that they can be cheaply produced in quantity. Hydrophobin-like substances can for example be isolated from nature or obtained from genetically modified organisms and purified according to Wessels and Wösten et al. (Wessels, J.G., 1997, Adv.Microb.Physiol 38:1-45; Wösten, H.A.B. et al., 1993, The Plant Cell 5:1567-1574) and modifications thereof. Before use, freeze-dried hydrophobin-like substance can disassembled with pure TFA and dried in a stream of nitrogen or filtered air. The monomeric protein can be dissolved in an aqueous solution such as 50 mM phosphate buffer or water.
As mentioned before, hydrophobins are among the most abundant proteins secreted by fungi. Class I hydrophobins appear to be the most promising for application because of the stability of the assembled films. These hydrophobins appear to be particularly abundant in the culture medium of members of the basidiomycetes. For instance, it has been calculated that, in 4-day-old cultures of *Schizophyllum commune*, about 15% of the ³⁵S incorporated into protein goes into synthesis of the SC3 hydrophobin while up to 20 mg of SC3 can be easily purified from one liter of culture medium by a simple procedure based on the extraordinary properties of the protein, dipping at hydrophobic/hydrophilic interfaces suffices to accumulate the hydrophobin -like substance. Strain selection, selecting strains yielding genetically modified hydrophobins and optimizing culture conditions may further enhance the yield as could molecular genetic methods, such as increasing gene dose and heterologous production in fungi in common use in the fermentation industry. On the other hand, it should be realized that quantities needed for certain applications are often small. This is easily realised from the use that nature makes of an "expensive" product as a protein for changing the wettability of surfaces. Indeed, the very nature of the assembled amphipathic film requires that it is present as a monolayer. A surface coated with a monolayer of hydrophobin-like substance need not be coated with a single monolayer only but can also be coated with multiple monolayers of said substance. The thickness of this single monolayer is only about 10 nm and thus very little hydrophobin is required to achieve a drastic change in wettability. For example, from the number of molecules of SC3 absorbed to Teflon, it can be calculated that about 1.5 mg SC3 hydrophobin suffices to coat 1 m² of Teflon surface with the effect of decreasing the hydrophobicity of this surface from 110° to 48° water contact angles.

Another important lesson from nature is that a fungal species makes different hydrophobins for different purposes. For instance, in S. *commune* an SC3 film appears to coat aerial hyphae and to confer water-repellent properties to these structures, whereas air channels in fruiting bodies are lined with an assemblage of SC4 hydrophobin. One wonders whether possible biophysical differences in the properties of these films are tuned to different functions, especially because hydrophobins from widely different species may be more closely related than different hydrophobins within one species. This is certainly a point to be considered in any biomimetics before resorting to genetic engineering to tailor a hydrophobin for a specific purpose.

For that matter, an object with a coated surface according to the invention for example finds its use in tissue engineering particularly for coating hydrophobic surfaces to increase their biocompatibility. As already noted, the attachment of the hydrophobin film to hydrophobic surfaces is very strong and the change in surface wettability significant. For instance an object with a coated surface according to the invention also finds its use to enhance the biocompatibility of medical implants, including artificial blood vessels and surgical instruments. Also, objects such as hydrophobic solids or liquids (oils) can be dispersed in water by coating with a hydrophobin. Oil vesicles coated with a hydrophobin film may for example be useful for delivery of lipophilic drugs. In short, the property of hydrophobins to coat a surface with a very thin layer (about 10 nm) that nevertheless dramatically changes the nature of this surface provides the use of these proteins in nanotechnology. Initially, monomeric forms are most water soluble, however some tetramers may be found in solution, in contrast to the assembled states (alpha helix-state or beta sheet-state).
Assemblage occurs at a wide range of temperatures, however for inclusion of reactive compound one should be guided by the individual sensitivity of such a compound to degradation upon heat. For example, most enzymes become instable at higher temperatures than 50 degrees Celsius; however, heat-stabile enzymes may also be used. Generally, coating improves over time and therefore it is provide to generally use about16 hours (i.e. overnight) to coat a surface. However, hydrophobin coating already occurs after 30 seconds, depending on the concentration of monomers in solution. Sufficient coating is obtained as long said solution is capable of forming the above described monolayer on the surface of said object by self-assembly of said compound. Such a s solution contains at least 100 nanogram, better 1 microgram, preferably 2 microgram, more preferably 20 to 100 microgram of hydrophobin-like compound per ml. Overdosing is generally only speeding up coating but not improving the coating per se. A method is preferred wherein said coated object is pretreated with hydrophobin-like substance prior to contacting said coated object with said reactive compound. It is also preferred that a pretreatment is selected which comprises contacting said coated object with a detergent solution, such as a Tween 20 (Polysorbate 20), NP40 (Nonidet P-40) or SDS solution. After coating with hycliophobin it is preferred to heat said coated object in said solution to 30-80 degrees Celsius. Alternatively, one selects alpha-helix state or beta-sheet state.

The invention also provides a method wherein, following immobilizing of a reactive compound, the object is subsequently coated with an amphipathic hydrophobin-like compound existing for at least 80%, better 90 or preferably 99% in the alpha-helical state, thereby providing a blocking method for array systems. In this way, the invention provides a method for obtaining an object, such as an array-system for probing with nucleic acid (be it DNA, RNA or PNA), or an assay system for immmunological detection (be it an Elisa plate or other surface at which binding interactions, such as between antibody and antigen, can take place) having at least part of its surface provided with a reactive compound wherein said surface is additionally provided with an amphipathic hydrophobin-like coating. It is also provided that such blocking consisting of a hydrophobin-like coating that essentially exists in the alpha-helical state. The invention also provides the use of a method as described herein in coating the surface of an object. Said surface can vary between a simple straight or an complicated curved surface, located at the inside or outside of said object. For example, also capillary activity of small tubes or microspheres or microsponges can be modulated by lining capillaries with a hydrophobin-like substance provided with a reactive compound according to the invention.

It is in particular herein provided to provide object surfaces with reactive molecules of proteinaceous nature, such molecule may comprise a conventional (poly- or monoclonal) or synthetic antibody or fragments thereof such as Fab or single chain type antibodies or other binding molecules (optionally additionally provided with an enzyme, such as a peroxidase or with a fluorescent group), an enzyme, such as glucose oxidase or cholesterol oxidase or peroxidase or mono-oxygenase as provided herein in the detailed description, or alkaline phosphatase, luciferase, esterase, lipase, or trypsin, or combinations of enzymes, a receptor for measuring ligand interaction, a light receptor or light harvesting complex, combinations thereof, and so on. Where covalent binding often results in loss of reactivity, these compounds bound with a method according to the invention in particular substantially maintain their reactivity towards ligands, antigens, substrates, etc, probably exactly because the bond with the coating is of a non-covalent nature. Such reactivity can for example be measured by surface plasmon resonance (SPR, also know as the BIACORE sensor system) which allows sensitive detection of molecular interactions in real time, without the use of labels. A gold object surface (hydrophobic) is coated with a hydrophobin solution by simply incubating for some time (half an hour is generally sufficient). Then the surface is washed with water to remove any unbound hydrophobin. The surface can be treated with detergent to obtain a β-sheet state coating. The surface can be used directly to bind small molecules (like ubiquinone), peptides, proteins enzymes, lipids, nucleic acids, etc. The mass increase can be detected and is a means of quantifying the amount of material bound to a certain surface area coated with hydrophobins.

The invention also provides an object having a surface that can be used for the detection of specific molecular interactions such as, for example, the detection of antibody-antigen interactions in display technologies or in an ELISA-type assay, or for the detection of nucleic acid-nucleic acid interactions, being it DNA, RNA or PNA. The amphipathic nature of hydrophobins makes them ideal blocking agents. Coating a hydrophobic surface with a hydrophobin-like substance reduces the "stickiness" of a hydrophobic surface and therefore decreases a-specific binding of hydrophobic compounds to hydrophobic areas.
This will improve the performance of such a detection method. The invention provides an object having at least part of the surface provided with a reactive compound wherein said surface is additionally provided with a hydrophobin-like compound to reduce unwanted a-specific interactions with said surface. In a preferred embodiment, a solid support surface, such as for example an ELISA plate, is coated with an antibody and thereafter the surface is further treated with a solution of monomeric hydrophobin, leaving the reactivity of said compound essentially unchanged and reducing the a-specific binding characteristics of said surface. In particular, the compound is a hydrophobic compound or a compound containing a hydrophobic anchor. Such compounds are among the compounds most stably maintained in the hydrophobin coating. It is thought that a planar hydrophobic compound or anchor may be beneficial. In the present application the term "anchor" is understood to mean a part of the compound, said part having a side and/or moiety lacking hydrophilic groups. It is also thought that the absence or a reduced number of negative and/or positive charges is advantageous. If charge is present, it is preferably from weakly acidic or basic groups, which can release or accept a hydrogenium ion to eliminate the charge.
The invention is exemplified further in the detailed description as provided herein.

### Detailed description

### 1. Immobilization of glucose oxidase on glassy carbon electrode

Glassy carbon electrode was coated with hydrophobin by placing the electrode in a solution of hydrophobin (100 µg/ml) and incubating for 15 minutes, after which the electrode was thoroughly rinsed with water. Subsequently, the coated electrode was submerged in a glucose oxidase containing solution (SIGMA, 210,000 units/g of solid, final concentration 1.8 mg/ml)) for 2 hours, and rinsed with water afterwards.
The electrode, modified and functionalized with the enzyme , was placed in a three electrode system, including an Ag/AgCl reference electrode and a Pt counter electrode, using phosphate buffer pH 7 (25 mM). When glucose was added, the immobilized glucose oxidase catalyzed the reaction leading to formation of hydrogen peroxide, which could be detected as a small current, which was proportional to the glucose concentration.. The glucose oxidase remained active upon immobilization on the hydrophobin layer.
The modified electrode was stored in a sealed container, not in liquid, at 4 °C, and tested frequently for activity and response to glucose. Over the period tested, 67 days, the electrode maintained its activity and was not influenced by the frequent testing.

### 2. Immobilization of cholesterol oxidase on glassy carbon electrode

Glassy carbon electrode was coated with hydrophobin by placing the electrode in a solution of hydrophobin (100 µg/ml) and incubating for 15 minutes, after which the electrode was thoroughly rinsed with water. Subsequently, the coated electrode was submerged in a cholesterol oxidase containing solution (2 mg/ml) for 2 hours, and rinsed with water afterwards. The electrode, modified and functionalized with the enzyme (cholesterol oxidase, 0.5U /ml), was placed in a three electrode system, including an Ag/AgCl reference electrode and a Pt counter electrode, using phosphate buffer pH 7 (25 mM) as electrolyte. To overcome the poor solubility of cholesterol in water, the cholesterol was solubilized in isopropanol with Triton in phosphate buffer (Ropers, M-H. et al., 2001, Phys. Chem. Chem. Phys. 3:240-245).
Upon addition of cholesterol, the immobilized cholesterol oxidase catalyzed the reaction leading to formation of hydrogen peroxide. The peroxide resulted in a small detectable current over the electrode, which was correlated to the cholesterol concentration added. This example demonstrated the immobilization of cholesterol oxidase, while remaining active.

### 3. Immobilization of glucose oxidase and peroxidase

Besides immobilizing on electrode surface, glucose oxidase was also immobilized on coated Teflon (polytetrafluorethylene PTFE) sheets. The coating was achieved by incubating the Teflon at 25 °C in 2 ml of a 100 µg/ml hydrophobin solution (here SC3, trSC3 and SC4 were used in separate experiments) in a 3 ml glass vial for 15 minutes. The sheets were removed from the hydrophobin solution and were washed with large amounts of water to remove any unbound hydrophobin.
The glucose oxidase was immobilized by incubating the coated Teflon sheets in a glucose oxidase containing solution (1 mg/ml) for 2 hours and rinsing with water afterwards.
The presence and activity of the immobilized glucose oxidase was tested by incubating the treated Teflon sheets in a solution with peroxidase (1520 units/mg, SIGMA; concentration 0.4 mg/ml) and o-Dianisidine (final concentration 68 µg/ml), which absorbs at 435 nm upon becoming oxidized and gives a visible color. Upon addition of glucose absorption at 435 nm was observed, which indicated the oxidation of o-dianisidine by peroxidase. The oxidation of o-dianisidine was directly related to the amount of peroxide formed by glucose oxidase, and therefore the glucose concentration.
Alternatively, the peroxidase was immobilized on Teflon and glucose oxidase was added in the solution. Coloring of the solution upon addition of glucose was an indication of the activity of the immobilized peroxidase.
Alternatively, glucose oxidase was immobilized on one sheet of Teflon, while peroxidase was immobilized on a different sheet of Teflon. Both sheets were placed in a container containing o-dianisidine and upon addition of glucose the solution became colored. This demonstrated the possibility to immobilize multiple enzymes which could catalyze a reaction involving multiple steps.
Removal of the coated and functionalized Teflon was sufficient to stop the reaction, indicating that the enzymes were not desorbed from the surface. The functionalized Teflon is still active after storage for several weeks at 4 °C.

### 4. Immobilization of different enzymes on Teflon surface.

Oxido-reductases (glucose oxidase, peroxidase, laccase), hydrolases (lipase, esterase) and a protease (tryspin) were immobilized via the SC3 hydrophobin on Teflon. The surface was first coated with SC3 hydrophobin by immersion in the protein aqueous solution (0.3 mg/mL) during approximately 30 minutes. The obtained modified surface was then immersed in an aqueous enzyme solution during approximately 30 minutes. After each step, the Teflon surface was rinsed with an adequate buffer solution. Activity of the immobilized enzymes was followed by spectrophotometry at 412 and 435 nm after immersion of the functionalized surface in the corresponding substrate solution. The optical density of the solution increases after the immersion of the functionalized surface and stays constant after its removal. The experiments showed that the immobilized enzymes are not denatured and are not desorbed from the surface.
The functionalized Teflon® was stored at 4 °C in an Eppendorf tube containing a buffer solution. The different surfaces are still functional after several weeks.

Enzymes used: glucose oxidase from *Aspergillus niger* (SIGMA; 210 U/mg of solid, final concentration 1.0 mg/mL), horseradish peroxidase (SIGMA; 1520 U/mg of solid, final concentration 0.14 mg/mL), laccase from *Trametes sp.* (Biocatalysts, Wales; final concentration 2.1 mg/mL), lipase from *Candida cylindracea* (Biocatalysts, Wales; final concentration 0.25 mg/mL), porcine liver esterase (SIGMA; 41 U/mg of solid, final concentration 2.4 mg/mL), bovine pancreas trypsin (SIGMA; 8,750 U/mg of solid).

Substrates: Glucose for glucose oxidase at pH 7; o-dianisidine for peroxidase and laccase in milli-Q water; p-nytrophenyl-caprylate for lipase at pH 7.7; p-nytrophenyl-acetate for esterase at pH 7.7 and N-a-benzoyl-d,l-arginine p-nitroanilide hydrochloride for trypsin.

### 5. Immobilization of antibody and second enzyme

Teflon sheets with a hydrophobin coating were prepared as described. The Teflon sheet was incubated in a solution of 100 µg/ml of equimolar amounts of glucose oxidase antibodies and peroxidase or with peroxidase alone for 2 hours at 25 °C.
The hydrophobin coated Teflon sheet with immobilized antibodies and/or peroxidase was washed extensively with water to remove all unbound protein.
The sheets were incubated for 1 hour in a solution with different (very dilute) concentrations of glucose oxidase at 4 °C. The sheets were washed with water to remove unbound enzyme and the sheet was placed in a.1.5 ml cuvette (parallel to the light beam) with 1 ml of peroxidase activity solution (4-aminophenazone and phenol). The reaction was started by the addition of glucose stock solution to the cuvette and followed by the formation of the color at 515 nm.
The Teflon sheet without the glucose oxidase antibodies showed hardly any conversion of glucose after incubation with the low concentrations of glucose oxidase, whereas the Teflon sheets with immobilized glucose oxidase antibodies after incubation with the same low concentrations of glucose oxidase was highly active. This example demonstrates that immobilized antibodies on a hydrophobin layer can be used to concentrate an enzyme from a highly diluted sample on a sensor surface.

### 6. Immobilization of antibodies

A microtiter plate well was incubated with a hydrophobin containing solution (100µg/ml) for 15 minutes and rinsed thoroughly with water. Subsequently a solution containing Oregon green ® 488 goat anti-rabbit antibodies (06381, Molecular Probes)(Ab1) was added to the well. After 15 minutes incubation the well was decanted and rinsed extensively with water. The immobilization of the antibodies was analyzed via fluorescence according to the protocol provided by the supplier of the Oregon green ® labeled antibodies and compared with the fluorescence of a non-coated well (absorption/emission maxima ~496/524 nm).

Saturation of antibody Ab1 immobilization was studied by titration of the antibody Ab1 concentration and intensity of the fluorescence.
The effect of immobilizing antibodies upon binding to the coated surface was studied with two other second antibodies, one (Ab2a), which does not interact with Ab1, and the other one (Ab2b) does. The wells were treated as described above and the wells with the maximum amount of immobilized antibody Ab1 were used, to avoid immobilization of Ab2 on the hydrophobin coating directly.
Antibody Ab2a and antibody Ab2b containing solutions were added in various concentrations and after 15 minutes incubation removed and the well was rinsed. Both antibodies Ab2a and Ab2b have absorption/emission maxima, which are different from the maxima of Ab1, and can therefore be detected independently from Ab1 by fluorescence.
Alternatively Living Colors™ antibody against green fluorescent protein (GFP) was immobilized in hydrophobin coated wells, as described above. GFP was added to the well and incubated for 15 minutes. After extensive rinsing the well remained fluorescent, indicating the immobilized (non-fluorescent) antibody remained its ability to bind the GFP. The negative control was the hydrophobin coated well, treated with the previous mentioned Oregon green 488 goat anti-rabbit antibodies, which did not immobilize GFP.
Alternatively, anti-galactosidase antibodies were immobilized on a hydrophobin coating, as described above, and β-galactosidase was added. After rinsing extensively the activity of the enzyine was tested by addition of X-gal. The negative control was the hydrophobin coated well, treated with the previous mentioned Oregon green 488 goat anti-rabbit antibodies, which did not immobilize galactosidase.
The effect of the confirmation of hydrophobin SC3 was studied by treating the wells, after incubation with hydrophobin SC3, with SDS at 80 °C for 1 hour. The same experiments were done as described above. Also the blocking effect of hydrophobins, added in their monomeric state, was studied, by titration of the amount of antibody Ab1 and additional monomeric hydrophobin SC3.
The same experiments were done following the standard ELISA protocol, demonstrating the improved sensitivity and ease of use of the non-covalent immobilization via hydrophobins.The standard ELISA protocol consisted of the immobilization of 100 µl anti-galactosidase antibody (10 µg/ml in 10 mM phosphate buffer pH 7.2), incubated for 3 hours at room temperature. The plate was emptied and residual liquid was tapped out. Washing was done with 0.1 M phosphate buffer, pH 7.2, containing 1 M NaCl and 0.02 % (v/v) Tween-20, and repeated 5 times. Blocking was done by addition of 300 µl blocking solution (BSA, skimmilk, and casein were tested) and incubation for 15 minutes. The wells were emptied, tapped out and washed 3 times. After blocking, a mixture with different concentrations of β-galactosidase were added to the different wells. After the washing the wells were tested for β-galactosidase activity as described before.This procedure was compared to the immobilization of anti-galactosidase antibodies on hydrophobin coated wells. Sensitivity of low concentrations of β-galactosidase was compared with the ELISA method.

### 7. Blocking effect of hydrophobins

The invention also provides an object having a surface that can be used for the detection of specific molecular interactions such as, for example, the detection of antibody-antigen interactions in display technologies or in an ELISA-type assay, or for the detection of nucleic acid-nucleic acid interactions, being it DNA, RNA or PNA. The amphipathic nature of hydrophobins makes them ideal blocking agents. Coating a hydrophobic surface with a hydrophobin-like substance reduces the "stickiness" of a hydrophobic surface and therefore decreases a-specific binding of hydrophobic compounds to hydrophobic areas.
This will improve the performance of such a detection method. The invention provides an object having at least part of the surface provided with a reactive compound wherein said surface is additionally provided with a hydrophobin-like compound to reduce unwanted a-specific interactions with said surface. In a preferred embodiment, a solid support surface, such as for example an ELISA plate, is coated with an antibody and thereafter the surface is further treated with a solution of monomeric hydrophobin, leaving the reactivity of said compound essentially unchanged and reducing the a-specific binding characteristics of said surface. We compared the blocking properties of hydrophobin with known blocking reagents as BSA, skim milk, casein and gelatin in a standard ELISA assay with slight modifications:
Wells of an ELISA plate were treated with a coating solution, containing 10 mM phosphate buffer pH 7.2 and 10 µg/ml anti-galactosidase antibody, incubated for one hour at room temperature and emptied by tapping out the liquid. Washing was done with 0.1 M phosphate buffer, pH 7.2, containing 1 M NaCl, and repeated 5 times. The blocking of the plate was done by adding several concentrations of BSA, skim milk, casein and monomeric hydrophobin, incubation for 15 minutes and emptied by tapping out the liquid and washed 3 times. After blocking, a mixture with different concentrations and ratios of β-galactosidase and GFP was added to the different wells, ranging from a 1000 fold excess β-galactosidase over GFP to a 1000 fold excess of GFP over β-galactosidase. The washing was done with 0.1 M phosphate buffer plus 1 M sodium chloride + 0.02 % (v/v) Tween-20 and repeated five times. After the washing the wells were tested for the presence of GFP by fluorescence, indicating the a-specific binding, and β-galactosidase activity as described before to test bincling of the protein of interest. The presence of GFP and β-galactosidase were compared with the type of blocking, indicating the superb blocking of hydrophobins.

### 8. Targeting through non-covalently bound ligands

Hydrophobins are thought to be versatile by genetically engineering construct, resulting in fusion proteins of hydrophobin and ligand. Alternatively, the fusion can happen after translation, by cross-linking. This example describes non-covalently bound ligands to hydrophobins, which even further increase the versatility and ease of use of hydrophobins.
The experiment was performed as described by Scholtmeijer et al (Scholtmeijer, K., et al., 2002, Appl. Environ. Microbiol 68: 1367-1373) with the modification of using non-modified hydrophobins which were being incubated after assembly with RGD-peptide containing solution. The growth and activity test of fibroblasts, and the rest of the example were done as described. Essentially, Teflon sheets were coated with hydrophobins as described and contacted with a solution containing a polypeptide comprising an amino acid sequence RGD (RGD-peptide) to immobilize the RGD-containing peptide on the coated surface. Fibroblasts (cell line L292, from mouse alveolar adipose tissue) were seeded at 7500 cells/cm² in wells of 24 well plates in which coated or bare Teflon sheets had been placed and containing RPMI 1690 medium supplemented with 10% foetal calf serum, 1 x 10⁴ u ml⁻¹ penicillin, 1 x 10⁴ u ml⁻¹ streptomycin and glutamine solution (1:100; Gibco BRL, USA). As a positive control, fibroblasts were grown in the absence of a Teflon sheet. Growth processes were evaluated at 24, 48, 72 and 96 h. Biocompatibility was assessed by microscopically estimating the confluence of the cultures at the various surfaces. The results indicated that non-covalent immobilization of a RGD-containing peptide can be used to create a modified surface and that this modification results in an increased biocompatibility.The results indicated that non-covalent interactions can also be used to create a modified surface.
Alternatively, hydrophobin vesicles were made as described by Wösten et al. (Wosten, H.A.B. et al., 1994, EMBO J. 13:5848-5854), using dansyl labeled trSC3, as described by Wang et al. (Wang, X. et al., 2002, Prot. Science. 11:1172-1181), allowing detection by fluorescence. The hydrophobin vesicles were incubated in a RGD-peptide containing solution, washed and then incubated with fibroblasts.
After separating the fibroblasts from the unbound hydrophobin vesicles, the presence of the vesicles in the cell-fiaction was determined by fluorescence and compared to the control of hydrophobin vesicles without the RGD sequence adhered.

### 9. Esterase, lipase, and trypsin immobilization

Colloidal Teflon (∅ 150 nm) and Teflon sheets, polyethylene sheets and glass sheets (0.8x2.5 cm) were incubated at 25 °C in 2 ml of a 100 µg/ml hydrophobin solution (either SC3, trSC3 or SC4) in a 3 ml glass vial for different time periods varying from 10 minutes to 16 hours. The sheets were removed from the hydrophobin solution and were immediately washed with large amounts of water to remove any unbound hydrophobin (5 x 50 ml water for 5 min); these coatings are referred to as the α-helix state. Part of the sheets was boiled in a 2% SDS solution for 10 min after which the sheets were extensively washed with water; these coatings are referred to as the β-sheet state. The colloidal Teflon was collected by gentle centrifugation (2 min, 2000 rpm), the supernatant was removed and the Teflon pellet was resuspended in 2 ml pure water followed by centrifugation. The Teflon pellet was washed 5 times with water to remove all unbound hydrophobin.
The bare and hydrophobin coated sheets (α-helix state and β-sheet state) were incubated in 2 ml of enzyme solution in 3 ml glass vials at 25 °C for 2 hours. The used enzyme solutions were 100 µg/ml of 60 kD esterase (porcine liver), 40 kD lipase (wheat germ), or 25 kD trypsin (bovine pancreas). The sheets were washed extensively with water to remove any unbound enzyme.
The sheets with and without immobilized enzyme were used in a chromogenic assay. A 1.5 ml cuvette was filled with 1 ml of a 5 mM 4-nitrophenyl acetate solution (esterase substrate), 5 mM Na-benzoyl-DL-arginine-p-nitroanilide (trypsin substrate), or 5 mM 1,2-di-O-decanoyl-rac-glycero-3-glutaric acid-resorufin labeled in the appropriate buffers. The cuvette was placed in the spectrophotometer and the reaction was started by placing the hydrophobin coated sheets with or without immobilized enzyme in the cuvette parallel to the light beam. The light absorbance at the appropriate wavelength was followed in time. After 1 hour of incubation the sheets were taken from the cuvette and the reaction in the cuvette was followed for an additional 24 hours. The sheets were washed with water and were transferred to a new cuvette with fresh substrate and the reaction was followed again. The sheets were incubated 3 times on the same day in fresh substrate, after 1 day, after 1 week and after 1 month to determine the stability of the coating by following the activity on the sheet and the remaining activity after removal of the sheet from the reaction mixture.
The results show that esterase, lipase and trypsin were attached to bare sheets as well as to hydrophobin coated sheets, both α-helix state and β-sheet state.
However, enzyme attached to bare sheets was not active, whereas the enzyme attached to the hydrophobin coated sheets was active and stable for up to 1 month in buffer or dry at 4 °C. Removal of the sheet from the substrate solution resulted in an absolute stop of the reaction indicating that the immobilized esterase, lipase, and trypsin are firmly attached to the sheets. The colloidal Teflon coated with hydrophobin was used for binding experiments of glucose oxidase, esterase, lipase and trypsin with circular dichroism spectrometry. 400 µl of a 100 µg/ml solution of the mentioned enzymes was added to a 1 mm cuvette and a circular dichroism spectrum was recorded between 190 and 250 nm. To this solution increasing concentrations of pure colloidal Teflon or hydrophobin coated Teflon was added and changes were followed by recording CD-spectra. Esterase, lipase, glucose oxidase and trypsin all underwent structural changes upon binding to bare Teflon as judged from differences in the CD-spectra of soluble enzyme with and without Teflon. When Teflon coated with hydrophobin are added to the enzyme solutions the changes in CD signal (after correction for the hydrophobin signal) are less pronounced. Also part of the enzyme can be removed from the solution by spinning down the colloidal Teflon, indicating that the enzyme immobilizes on the Teflon spheres. Furthermore, oil droplets were coated with hydrophobin. Coating of oil droplets was achieved by emulsifying 100 µl of oil (mineral oil or paraffin oil) in 3 ml of water by sonication and adding 3 ml of an aqueous solution of hydrophobin (200 µg/ml). Alternatively, the oil was directly emulsified in the hydrophobin solution and 3 ml was added. After overnight incubation at room temperature or 60°C in the presence of 0.02% NaN₃, the emulsions were centrifuged at 10,000×g for 30 min and the oil droplets washed four times with water by centrifugation to remove soluble hydrophobin.
Oil droplets of assembled hydrophobin were then resuspended in a small volume of water (total volume 200 µl).
20 µl of oil droplets coated with hydrophobin were incubated in 1 ml of 100 µg/ml of enzyme solutions of esterase, lipase and trypsin for two hours at 25 °C. After incubation the oil droplets were washed 5 times with water by centrifugation as described above to remove all unbound enzyme. The washed SC3 coated oil droplets with enzyme bound to it were transferred to 1.5 ml cuvettes with 1 ml of the appropriate substrate solutions as described above for Teflon, polyethylene and glass sheets. The reaction was followed spectrophotometrically for 1 hour and the oil droplets were pipetted out of the cuvette and the remaining solution was followed for another 24 hours.
The results indicate that lipase, esterase and trypsin can be immobilized on SC3 coated oil droplets and that even after extensive washing steps the enzyme remains attached. The immobilized enzyme is still catalytically active as judged from the conversion of substrate in the cuvette as soon as the coated oil droplets are added. Removal of the oil droplets kills the conversion, indicating that all enzymes are efficiently removed by removing the oil droplets.

### 10. Immobilization of fire fly luciferase:

150 µl of a 100 µg/ml hydrophobin solution (either SC3, TrSC3 or SC4) or 150 µl water was added to wells of 2 microtiter plates. This was incubated at 25 °C for different time periods varying from 10 minutes to 16 hours. The wells were washed extensively with water to remove any unbound hydrophobin; these coatings were referred to as the α-helix state. A hot 2% SDS-solution was added to the hydrophobin coated and uncoated wells of one microtiter plate and this plate was placed at 60°C for 20 min. The SDS-treated wells were extensively washed with water. These coatings were referred to as the β-sheet state.
150 µl of 10-100 µg/ml of firefly luciferase (Roche, cat no. 411523) solutions were added to the bare and hydrophobin coated wells (α-helix and β-sheet) and incubated at 0 °C for 2-16 hours. The wells were washed extensively with 0.5 M Tris-acetate buffer, pH 7.5 to remove any unbound enzyme. To assay the activity of the immobilized enzyme 100 µl of luciferase reagent in 0.5 M Tris-acetate buffer, pH 7.5 (the substrate, Roche, ATP Bioluminescence assay kit CLS II, bottle 1, Cat. No. 1699 695) was added to the firefly luciferase coated wells. The reaction was started by adding 50 µl of a dilution series (3x10⁻⁵ to 3x10⁻¹⁰ M) of an ATP stock solution (Roche, ATP Bioluminescence assay kit CLS II, bottle 2, Cat. No. 1 699 695) to the wells. Luminescence was measured and followed in time using a MTP-format luminometer according to protocol.
After 1 hour of incubation the substrate solution was removed from the wells and the wells were washed three times with 0.5 M Tris-acetate buffer, pH 7.5. Fresh substrate and ATP solutions were added to the wells and the reaction was followed again. The wells were incubated 3 times on the same day in fresh substrate, after 1 day, after 1 week and after 1 month to determine the stability of the coating by following the activity in the wells.
The results show that firefly luciferase is attached to both bare and hydrophobin coated wells (α-helix state and β-sheet state). However, enzyme attached to bare wells was not active, whereas the enzyme attached to the hydrophobin coated wells was active and stable for up to 1 month

(NOTE: the concentrated luciferase solution of 1 mg/ml is stable for several months when frozen in 50µl portions, diluted solutions are unstable and should be kept in an ice-bath! It is also noted that it is sensitive to shaking and to storage in plastic vessels!)

### 11. Immobilization of alkaline phosphatase:

150 µl of a 100 µg/ml hydrophobin solution (either SC3, TrSC3 or SC4) or 150 µl water was added to wells of 2 microtiter plates. This was incubated at 25°C for different time periods varying from 10 minutes to 16 hours. The wells were washed extensively with water to remove any unbound hydrophobin; these coatings were referred to as the α-helix state. A hot 2% SDS-solution was added to the hydrophobin coated and uncoated wells of one microtiter plate and this plate was placed at 60°C for 20 min. The SDS-treated wells were extensively washed with water. These coatings were referred to as the β-sheet state.
A dilution series of 5 to 100 times diluted human placental alkaline phosphatase (4 u/ml, Roche, SEAP chemiluminescent Reporter gene assay, Cat no. 1 779 842) in the supplied dilution buffer was made. 150 µl of each dilution was added to the bare and hydrophobin coated wells (α-helix state and β-sheet state) and incubated at 25 °C for 2 hours. The wells were washed extensively with dilution buffer to remove any unbound enzyme.
To assay the activity of the immobilized enzyme 50 µl of substrate reagent and 100 µl dilution buffer (Roche, SEAP chemiluminescent Reporter gene assay, Cat no. 1 779 842) was added to the alkaline phosphatase coated wells and incubated at 25°C for 10 min. Luminescence was measured and followed in time using a MTP-format luminometer according to protocol.
After 2 hours of incubation the substrate solution was removed from the wells and the wells were washed with dilution buffer. Fresh substrate and dilution buffer was added to the wells and the reaction was followed again. The wells were incubated 2 times on the same day in fresh substrate, after 1 day, after 1 week and after 1 month to determine the stability of the coating by following the activity in the wells.
The results show that alkaline phosphatase is attached to both bare and hydrophobin coated wells (α-helix state and β-sheet state). However, enzyme attached to bare wells was not active, whereas the enzyme attached to the hydrophobin coated wells was active and stable for up to 1 month.

### 12. Immobilization of Light Harvesting complex (LHC):

Three different electrode substrates (hydrophilic GE and hydrophobic GCE or TMFE) were coated with hydrophobin (either SC3, TrSC3 or SC4) in a 100 µg/ml hydrophobin solution and 2 were placed in water. This was incubated at 25 °C for different time periods varying from 10 minutes to 16 hours. The electrodes were washed with water to remove any unbound hydrophobin; these coatings were referred to as the α-helix state. One hydrophobin coated and one bare electrode of each type was boiled in 2% SDS-solution for 10 min. The SDS-treated electrodes were extensively washed with water. These coatings were referred to as the β-sheet state. The various types of coated and bare electrodes were loaded with electroactive compounds Q10, azobenzene or Q0 as described by Bilewicz et al in J. Phys. Chem. B 2001, 105, 9772-9777 or with a mediator such as methylene blue. The various types of coated and bare electrodes either or not loaded with the electroactive compounds were incubated in LHC of *Cyclotella cryptica,* isolated as indicated in Rhiel et al. (Rhiel E. et al., 1997, Botanica Acta 110, 109-117), at various concentrations for 2 h at 25°C. The electrodes were washed with the appropriate buffers.
To assay the activity of the immobilized LHC, the electrodes (in the appropriate buffer) were placed in the dark followed by placing them in daylight and measuring the current. The dark-light cycles were repeated several times on the same day, after 1 day, after 1 week and after 1 month to determine the stability of the immobilized LHC.

### 13. Immobilizing DNA

Besides proteins, also other molecules can be immobilized. As an example the immobilization of DNA is described.
Though there are well known methods to immobilize DNA, most methods to immobilize DNA are based on covalent binding and therefore require a reactive group, usually the 5' end of the DNA molecule. Immobilization of DNA on a hydrophobin coating is also firm, but does not require a reactive end of the DNA, and is therefore more versatile. At the same time, the immobilization is less sensitive to breakage, compared to the linear covalently bound DNA. This example describes the immobilization of DNA and the accessibility upon binding.

PCR tubes (polypropylene, Sarstedt) were coated at 25 °C for 16 hours with 50 µl of a 100 µg/ml SC3 hydrophobin solution. The SC3 hydrophobin solution was removed and 100 µl of 1 % SDS was added and heated to 80 °C for 10 minutes, this results in B-sheet formation. Alternatively 100 µl water was added and also heated for 10 minutes at 80°C (α-helical state). Tubes were rinsed 5 times with 100 µl water and
A plasmid containing solution was added and incubated for 15 minutes, after which the unbound DNA was rinsed away with 10 times 200 µl water. The tubes with the immobilized DNA were used as reaction tubes in a PCR reaction with primes specific for the immobilized DNA. A standard PCR was performed in the tubes (50 µl, 1' 96 °C, 30" 60 °C, 4' 72 °C, 25 cycles) and the product was analyzed on gel. The non-coated tubes did not result in a PCR product, indicating the DNA was rinsed away. The tubes with the α-helical coating did result in PCR product, demonstrating the immobilization of the plasmid DNA on the hydrophobin coating. The tubes with the β-sheet coating did not result in DNA immobilization, indicating different specificity between different coatings.
The resulting PCR product does not only indicate the ability of hycliophobin to immobilize DNA, it also indicates the immobilized DNA is still accessible for oligonucleotides and enzymes like polymerase.

### 14. Immobilization of CYP2D6 and CYP2C19 on Teflon surface

Cytochrome P450 is a superfamily of heme-containing mono-oxygenases. They contain an iron atom at the active site, which can bind in the oxidized form (Fe3+) the substrate. Reduction takes place and Fe2+ is being formed. With molecular oxygen the Fe2+ can be oxidized to Fe3+ again, upon release of the now oxidized product. Two examples of cytochrome P450 are CYP2D6 and CYP2C19, located in the human liver and involved in metabolizing xenobiotics like drugs.

The cytochromes CYP2D6 and CYP2C19 were immobilized on a Teflon sheet, by incubating the Teflon in a solution with hydrophobin (100 µg/ml), as described above, and after rinsing in a solution with the cytochromes and finally rinsed again. The Teflon sheets coated with CYP2D6 and CYP2C19 bound on the Teflon were incubated in a medium containing NADPH and the model substrates dextromethorphan and mephenytoin. After incubation for several time periods the substrates dextromethorphan and mephenytoin were measured together with their metabolites dextrorphan and 4-hydroxymephenytoin. The degree of metabolism of either immobilized cytochrome was calculated from the ratio substrate to product.

### 15. Immobilization of CYP2D6 and CYP2C19 on an electrode surface

A glassy carbon electrode was coated with hydrophobin by emerging the electrode for 15 min in a solution containing hydrophobin (100 µg/ml). The electrode was thereafter thoroughly rinsed.The cytochromes CYP2D6 and CYP2C19 were separately bound on one hydrophobin coated electrode each, by incubation CYP2D6 or CYP2C19 containing solution for 15 minutes and extensively rinsed.The modified electrodes were placed in a medium containing NADPH and the model substrates dextromethorphan and mephenytoin. During the incubation period the potential was measured, which was induced by the contact with the substrates dextromethropan and mephenytoin. The magnitude of the potential reflects the metabolism by the iso-enzymes.After the incubation for 1 hour, the electrodes were removed and the substrates (dextromethorphan and mephenytoin, respectively) and products (dextrorphan and 4-hydroxymephenytoin, respectively) were quantified. The ratio of both was a reflection of the activity of the cytochromes and could be correlated to the measured potential.

### 16. Immobilization of BSA, fibronectin or proteins present in RPMI-medium

BSA (bovine serum albumin) and fibronectin were immobilized on coated Teflon sheets. The coating was achieved by incubating the Teflon at 25 oC in 2 ml of a 100 µg/ml hydrophobin solution (either SC3 or trSC3) in a 3 ml glass vial for 15 minutes. The sheets were removed from the hydrophobin solution and were immediately washed with large amounts of water to remove any unbound hydrophobin. Coated and bare Teflon sheets were incubated in either a BSA or fibronectin containing solution (50 µg/ml) for 14 hours and were rinsed with water afterwards. The presence of BSA or fibronectin on bare and coated Teflon was detected extracting all protein with TFA, evaporating the TFA and loading the sample on SDS-PAGE and staining according to standard procedures.
This demonstrated that BSA and fibronectin were not immobilized on bare Teflon while they can be immobilized on coated Teflon. Furthermore, different quantities of BSA or fibronectin were present on the SC3 coated and the TrSC3 coated Teflon sheets. In addition, a similar experiment as described above was performed using supplemented RPMI-medium (Sigma Cat no. R 0883) instead of BSA or fibronectin. Here, also no immobilization of proteins could be observed on bare Teflon while differences in the quantity of proteins could be detected on SC3 or TrSC3 coated Teflon.

### 17. Use in a biosensor system.

Another application of the invention relates to a method of providing a sensor type possessing favourable features for use as a biosensor. Biosensors use biological molecules to detect other biological molecules or chemical substances. Biosensors might for example use a monoclonal antibody to detect an antigen, or a small synthetic DNA molecule to detect DNA. A biosensor is a device that incorporates a biological recognition (sensing) element in close proximity or integrated with the signal transducer, to give a reagentless sensing system specific to a target compound (analyte). Transducers are the physical components of the sensor that respond to the products of the biosensing process, which may be optical, electrochemical, thermometric, piezoelectric or magnetic, and outputs the response in a form that can be amplified, stored, or displayed. The biological recognition element may be a biological material or a biomimic (e.g. tissue, microorganisms, organelles, cell receptors, enzymes, antibodies, nucleic acids etc.). The advantage of biological sensing elements is their remarkable ability to distinguish between the analyte of interest and similar substances. Biosensing occurs only when the analyte is recognized specifically by the biological element. With biosensors, it is possible to measure specific analytes with great accuracy.
It is preferred that the biological elements are bound to the sensor surface in a non-covalent manner, as is the case in the method provided in the invention, leaving secondary and tertiary structures of such biological compounds virtually intact and thus allowing optimal biological recognition.

For a given analyte-recognition element reaction, several transduction schemes may be applicable. Amperometric devices detect changes in current as constant potential. Conductimetric devices detect changes in conductivity between two electrodes. Potentiometric devices detect changes in potential at constant current (usually zero). Optical transducers can be subdivided into two modes (extrinsic and intrinsic) according to the optical configuration. In the intrinsic mode, the incident wave is not directed through the bulk sample, but propagates along a wave guide and interacts with the sample at the surface within the evanescent field. Other surface methods of optical detection of biological recognition are based on modulation of the field excited at the interface between different materials due to incident light. For example, the BIAcore system monitors biospecific-interactions with a surface plasmon resonance detector. The mass detector is generally based on a quartz crystal whose frequency is predictably affected by minor changes at the surface such as antibody binding to a surface-immobilized antigen.
The realization of the biosensor concept is in most cases dependent on key technologies that enable component fabrication and assembly in the 'reagentless' form. The reagentless form in most cases is achieved by immobilizing the biological recognition element onto the sensor surface. Advantageously, the biosensor surface is coated with a compound allowing non-covalent attachment of a biological recognition molecule. For example, a gold sensor surface (hydrophobic) is coated with a hydrophobin solution by simply incubating for some time. Then the surface is washed with water to remove any unbound hydrophobin. Following treatment of the surface with detergent to obtain a beta-sheet state coating, the coated surface is used directly to bind a biological recognition molecule like antibodies, enzymes, peptides, lipids, nucleic acids or carbohydrates. In general, the immobilization matrix may function purely as a support, or else it may also be concerned with mediation of the signal associated with recognition of the analyte by the biological sensing element.

It is preferred that the immobilization matrix does not interfere with the sensitivity of the biosensor. Whereas the mere coating of a sensor surface with hydrophobin would result in reduced signal transmission, we found that it is possible to incorporate a compound in said hydrophobin coating to increase the sensor performance. For example, as provided herein, such a compound is an electroactive compound that is incorporated in the hydrophobin coating to improve the sensitivity of the sensor in comparison with a hydrophobin coating not containing said compound. In a preferred embodiment, the invention provides a method of providing a sensor surface with a hydrophobin-like substance wherein said coating is additionally provided with a reactive compound to improve the sensitivity of the sensor, also referred to as signal transducer, as well as with a non-covalently bound biological recognition compound.

## Claims

1. An object having at least a part of its surface provided with a hydrophobin-like coating (or membrane) wherein said coating is additionally provided with a reactive compound that is non-covalently attached to said coating and wherein said reactive compound is an antibody, an enzyme, a peptide, a lipid, a nucleic acid or a carbohydrate.

2. An object according to claim 1 wherein said reactive compound has a molecular weight (MW) larger than 1 kilodalton (1 kD).

3. An object according to anyone of claims 1 to 2 wherein said coating is essentially devoid of mannose residues.

4. An object according to anyone of claims 1 to 3 wherein said reactive compound comprises an enzyme.

5. An object according to anyone of claims 1 to 3 wherein said reactive compound comprises an antibody or a fragment thereof.

6. An object according to anyone of claims 1-5 wherein said surface comprises a liquid/liquid interface.

7. An object according to anyone of claims 1-5 wherein said surface comprises a solid/liquid interface.

8. A method of providing a surface of an object with a reactive compound, wherein said reactive compound is an antibody, an enzyme, a peptide, a lipid, a nucleic acid or a carbohydrate, comprising the steps of coating at least a part of the surface of the object with a coating of a hydrophobin-like substance and contacting the reactive compound with the coated hydrophobin-like substance to form a non-covalent bond between said hydrophobin-like substance and said compound.

9. A method according to claim 8 wherein said substance is capable of forming a monolayer on the surface of said object by self-assembly of the substance.

10. A method according to claim 8 or 9 wherein said coated object is pretreated prior to contacting said coated object with said reactive compound.

11. A method according to claim 10 wherein said coated object is pretreated with a detergent solution.

12. A method according to claim 8 to 11 comprising heating the surface of said coated object to a temperature ranging between 30 to 80 degrees Celsius.

13. A method of providing a surface of an object with a reactive compound comprising the steps of coating at least a part of the surface of the object with a coating of a reactive compound and subsequently contacting the surface with a solution of a hydrophobin-like substance, leaving the reactivity of said compound essentially unchanged.

## Patentansprüche

1. Objekt, dessen Oberfläche mindestens teilweise mit einer Hydrophobin-ähnlichen Beschichtung (oder Membran) versehen wurde, wobei die Beschichtung zusätzlich eine reaktive Verbindung besitzt, die nicht-kovalent an die Beschichtung gebunden ist und wobei die reaktive Verbindung ein Antikörper, ein Enzym, ein Peptid, ein Lipid, eine Nucleinsäure oder ein Kohlenhydrat ist.

2. Objekt gemäß Anspruch 1, wobei die reaktive Verbindung ein Molekulargewicht (MG) von mehr als 1 Kilodalton (1 kD) hat.

3. Objekt gemäß einem der Anspruche 1 bis 2, wobei die Beschichtung im Wesentlichen frei von Mannoseresten ist.

4. Objekt gemäß einem der Ansprüche 1 bis 3, wobei die reaktive Verbindung ein Enzym umfasst.

5. Objekt gemäß einem der Ansprüche 1 bis 3, wobei die reaktive Verbindung einen Antikörper oder ein Fragment davon umfasst.

6. Objekt gemäß einem der Ansprüche 1 bis 5, wobei die Oberfläche eine flüssig/flüssig- Grenzfläche umfasst.

7. Objekt gemäß einem der Ansprüche 1 bis 5, wobei die Oberfläche eine fest/flüssig- Grenzfläche umfasst.

8. Verfahren zur Herstellung einer Oberfläche eines Objekts mit einer reaktiven Verbindung, wobei die reaktive Verbindung ein Antikörper, ein Enzym, ein Peptid, ein Lipid, eine Nucleinsäure oder ein Kohlenhydrat ist, umfassend die Schritte des Beschichtens von mindestens einem Teil der Oberfläche des Objekts mit einer Beschichtung aus einem Hydrophobin-ähnlichen Stoff und des Inkontaktbringens der reaktiven Verbindung mit dem beschichteten Hydrophobin-ähnlichen Stoff zum Bilden einer nicht-kovalenten Bindung zwischen dem Hydrophobin-ähnlichen Stoff und der Verbindung.

9. Verfahren gemäß Anspruch 8, wobei der Stoff in der Lage ist, eine Monoschicht auf der Oberfläche des Objekts durch Selbstzusammensetzung des Stoffes zu bilden.

10. Verfahren gemäß Anspruch 8 oder 9, wobei das beschichtete Objekt vor dem Inkontaktbringen mit dem beschichteten Objekt mit der reaktiven Verbindung vorbehandelt wird.

11. Verfahren gemäß Anspruch 10, wobei das beschichtete Objekt mit einer Detergenzlösung vorbehandelt wird.

12. Verfahren gemäß Anspruch 8 bis 11, umfassend das Erhitzen der Oberfläche des beschichteten Objekts auf eine Temperatur im Bereich zwischen 30 bis 80 °C.

13. Verfahren zum Versehen einer Oberfläche eines Objektes mit einer reaktiven Verbindung, umfassend die Schritte des Beschichtens von mindestens einem Teil der Oberfläche des Objekts mit einer Beschichtung aus einer reaktiven Verbindung und nachfolgend des Inkontaktbringens der Oberfläche mit einer Lösung eines Hydrophobin-ähnlichen Stoffes, wobei die Reaktivität der Verbindung im Wesentlichen unverändert bleibt.

## Revendications

1. Objet ayant au moins une partie de sa surface munie d'un revêtement (ou membrane) de type hydrophobine, dans lequel ledit revêtement est muni de plus d'un composé réactif qui est attaché de manière non-covalente audit revêtement et dans lequel ledit composé réactif est un anticorps, une enzyme, un peptide, un lipide, un acide nucléique ou un hydrate de carbone.

2. Objet selon la revendication 1, dans lequel ledit composé réactif a un poids moléculaire (PM) supérieur à 1 kilodalton (1 kD).

3. Objet selon l'une quelconque des revendications 1 à 2, dans lequel ledit revêtement est essentiellement dépourvu de résidus de mannose.

4. Objet selon l'une quelconque des revendications 1 à 3, dans lequel ledit composé réactif comporte une enzyme.

5. Objet selon l'une quelconque des revendications 1 à 3, dans lequel ledit composé réactif comporte un anticorps ou un fragment de celui-ci.

6. Objet selon l'une quelconque des revendications 1 à 5, dans lequel ladite surface comporte une interface liquide/liquide.

7. Objet selon l'une quelconque des revendications 1 à 5, dans lequel ladite surface comporte une interface solide/liquide.

8. Procédé pour munir une surface d'un objet d'un composé réactif, dans lequel ledit composé réactif est un anticorps, une enzyme, un peptide, un lipide, un acide nucléique ou un hydrate de carbone, comportant les étapes consistant à revêtir au moins une partie de la surface de l'objet à l'aide d'un revêtement d'une substance de type hydrophobine et à mettre en contact le composé réactif avec la substance de type hydrophobine revêtue pour former une liaison non-covalente entre ladite substance de type hydrophobine et ledit composé.

9. Procédé selon la revendication 8, dans lequel ladite substance est capable de former une monocouche sur la surface dudit objet par un auto-assemblage de la substance.

10. Procédé selon la revendication 8 ou 9, dans lequel ledit objet revêtu est prétraité avant contact dudit objet revêtu avec ledit composé réactif.

11. Procédé selon la revendication 10, dans lequel ledit objet revêtu est prétraité à l'aide d'une solution détergente.

12. Procédé selon les revendications 8 à 11, comportant le chauffage de la surface dudit objet revêtu jusqu'à une température dans la plage comprise entre 30 et 80 degrés Celsius.

13. Procédé pour munir une surface d'un objet d'un composé réactif comportant les étapes consistant à revêtir au moins une partie de la surface de l'objet à l'aide d'un revêtement d'un composé réactif et à mettre en contact par la suite la surface avec une solution d'une substance de type hydrophobine, en laissant la réactivité dudit composé essentiellement inchangée.
